# EUROPÄISCHE PATENTANMELDUNG

(11) **EP 2 177 114 A1**
(43) Veröffentlichungstag der Anmeldung: **21.04.2010**
(21) Anmeldenummer: 09012945.3
(22) Anmeldetag: 13.10.2009
(51) Int. Cl.: A23L 1/30, A61K 36/185

(54) **Zusammensetzung für ein Nahrungsergänzungsmittel mit Zusatz von Heisteria Pallida**

(30) Priorität: 16.10.2008 DE 102008051906
(71) Anmelder: Wiemann, Wolfram, Dr., 90455 Nürnberg (DE)
(72) Erfinder: Wiemann, Wolfram, Dr., 90455 Nürnberg (DE)
(74) Vertreter: Stippl, Hubert

(57) **Zusammenfassung**

Die Erfindung betrifft eine Zusammensetzung für ein Nahrungsergänzungsmittel, insbesondere Sportler-Nahrungsergänzungsmittel, in flüssiger oder fester Form zur kontinuierlichen oder diskontinuierlichen Einnahme, wobei es als Bestandteil Heisteria Pallida enthält. Zur Lösung der erfindungsgemäßen Aufgabe, eine Zusammensetzung für ein Nahrungsergänzungsmittel zur Verfügung zu stellen, welche durch entzündliche Prozesse wegen sportlicher Dauerbelastung begründete Leistungseinbrüche weitestgehend verhindert, wird eine Zusammensetzung für ein Nahrungsergänzungsmittel vorgeschlagen, welches Heisteria Pallida enthält.

## Beschreibung

Die vorliegende Erfindung betrifft eine Zusammensetzung für ein Nahrungsergänzungsmittel in fester oder flüssiger Form zur kontinuierlichen oder diskontinuierlichen Einnahme, insbesondere ein entsprechendes Nahrungsergänzungsmittel oder diätetisches Supplement für Sportler.

Es ist bekannt, dass Sportler insbesondere vor und/oder während der sportlichen Betätigung Nahrungsergänzungsmittel zu sich nehmen, um dem durch die sportliche Betätigung belasteten Organismus insbesondere Mineralstoffe sowie Vitamine zukommen zu lassen. Gerade bei einer sportlichen Überbelastung, beispielsweise der Kniegelenke bei einem Marathonlauf, werden durch die bisher bekannten Nahrungsergänzungsmittel dem Sportler zwar Mineralien und Vitamine zugeführt, welche allerdings nicht verhindern können, dass es zu entzündlichen Prozessen aufgrund der sportbedingten Überbelastung kommt.

Die Aufgabe der vorliegenden Erfindung besteht darin, eine neuartige Zusammensetzung für ein Nahrungsergänzungsmittel zur Verfügung zu stellen, welches einen durch entzündliche Prozesse wegen einer sportlichen Dauerbelastung begründeten Leistungseinbruch weitestgehend verhindert.

Die vorstehende Aufgabe wird durch eine Zusammensetzung für ein Nahrungsergänzungsmittel gelöst, welche darin gekennzeichnet ist, dass sie als Bestandteil Heisteria Pallida enthält. Untersuchungen haben ergeben, dass bei Einnahme von Heisteria Pallida vor der sportlichen Betätigung entzündlich bedingte Schmerzen, insbesondere im Kniebereich, weitestgehend ausgeschlossen werden können. Der Sportler bleibt im Vergleich zu bisher bekannten Nahrungsergänzungsmitteln für Sportler wesentlich länger während der sportlichen Betätigung leistungsfähig. Darüber hinaus wird die oxidative Belastung und potentielle Schädigung der Gelenke durch die sportliche Betätigung aufgrund der besonderen Wirkung von Heisteria Pallida erheblich minimiert.

Bei der Angabe Heisteria Pallida handelt es sich um eine Pflanze, die in tropischen sowie subtropischen, feuchten Gegenden, wie z. B. Südamerika vorkommt.

Zweckmäßigerweise liegt Heisteria Pallida in der beanspruchten Zusammensetzung als vermahlenes Pulver der Rinde der vorgenannten Pflanze vor. Diese Anwendung ist zum einen besonders wirksam, zum anderen lässt sie sich technologisch einfach umsetzen.

Alternativ kann Heisteria Pallida auch als Extrakt vorliegen.

Gemäß einer zweckmäßigen Ausgestaltung umfasst die Zusammensetzung zusätzlich mindestens ein Vitamin, insbesondere Vitamin C. Die Zugabe von Vitaminen, insbesondere Vitamin C, sorgt für einen Grundstock mit antioxidativen Wirkungen. Zusätzlich zu Vitamin C kann auch Vitamin E, Zink, Selen sowie weitere antioxidative Nährstoffe bzw. Sekundärstoffe (z.B. Q10, Alpha-Liponsäure) vorgesehen sein.

Die Darreichungsform der Zusammensetzung weist zweckmäßigerweise eine definierte Dosierung von Heisteria Pallida auf. Zweckmäßigerweise wird die Zusammensetzung in Form einer Tablette oder Kapsel appliziert.

Hierzu kann die betreffende Tablette oder Kapsel in einem Blister als Verkaufseinheit vorgesehen sein.

Alternativ kann die Zusammensetzung auch als Trinkampulle, loses Pulver (insbesondere mit Messlöffel oder in Portionsbeuteln) oder in Form eines Riegels verwendet werden.

Ein Anteil von mindestens 100 mg pro Verabreichungseinheit, vorzugsweise ein Anteil von 100 mg bis 2000 mg, besonders vorzugsweise 500 mg bis 1500 mg an Heisteria Pallida hat sich als ganz besonders brauchbar erwiesen.

Was die Zerkleinerung der Rinde anbelangt, weist diese in einer besonderen Ausgestaltung der erfindungsgemäßen Zusammensetzung einen Feinheitsgrad im Bereich von 100 nm bis 1000 nm auf.

Ferner ist je Verabreichungseinheit mindestens ein Anteil von 100 mg, vorzugsweise ein Anteil von 100 mg bis 1000 mg an Vitamin vorgesehen.

Zweckmäßigerweise wird die erfindungsgemäße Zusammensetzung derart appliziert, dass bei der Anwendung durch Sportler eine diskontinuierliche Einnahme in einem definierten Zeitfenster vor der körperlichen Betätigung vorgesehen ist.

Die vorliegende Erfindung betrifft darüber hinaus die Verwendung von Heisteria Pallida als Bestandteil einer Zusammensetzung für ein Mittel, insbesondere für ein Nahrungsergänzungsmittel zur Steigerung der körperlichen Leistungsfähigkeit, insbesondere während der sportlichen Betätigung.

Die Einnahme von Heisteria Pallida vor der sportlichen Betätigung soll in fest vorgesehenen Dosierungen erfolgen.

Da die Zusammensetzung Heisteria Pallida enthält und die Zusammensetzung vor der körperlichen Tätigkeit in entsprechend vorgegebenen Dosierungen innerhalb eines Zeitfensters vom Sportler eingenommen wird, wird der Organismus des Sportlers vor Beginn der sportlichen Tätigkeit in einen erhöhten antioxidativen Status versetzt. Dieser erhöhte antioxidative Status wirkt gleichsam wie ein Puffer für während der sportlichen Betätigung gebildete freie Radikale. Diese werden aufgrund des Vorhandenseins von Heisteria Pallida eingefangen bzw. neutralisiert und verursachen daher auch bei längerer sportlicher Betätigung, wie z. B. bei einem Marathon, keine leistungsreduzierenden Entzündungen im Bereich der Gelenke oder im Muskelbereich.

Aufgrund dessen kann ein Sportler in Sportarten mit hohen Gelenksbelastungen (z.B. Kraftsport, Laufsport) oder erhöhtem Risiko von Hautreizungen (z.B. Radsport) durch Einnahme der erfindungsgemäßen Zusammensetzung im Vergleich zu bisherigen Zusammensetzungen des Standes der Technik über einen längeren Zeitraum hohe sportliche Leistungen erbringen. Nebenwirkungen sind unbekannt.

Nachstehend wird ein Zusammensetzungsbeispiel einer Kautablette als Nahrungsergänzungsmittel für einen Sportler wiedergegeben.

| Zutaten: | |
|---|---|
| Füllstoff z. B. Sorbit | 2,0 g |
| Heisteria Pallida gemahlen | 1,5 g |
| Vitamin C | 1,0 g |

Eine entsprechende Tablette ist von einem Sportler in fest vorgegebenen Abständen vor der sportlichen Betätigung sozusagen in einem fest vorgegebenen Einnahmeplan einzunehmen.

Des Weiteren wird nachstehend ein Zusammensetzungsbeispiel eines Riegels als Nahrungsergänzungsmittel für einen Sportler wiedergegeben.

| Zutaten: | |
|---|---|
| Füllstoff z. B. Schokoladenwaffelmasse bzw. Müsliriegelmasse | 30 g |
| Heisteria Pallida | 1,0 g |
| Vitamin E (50 %) | 0,2 g |
| Mineralien z. B. Magnesiumcitrat | 1,0 g |

Je nach Anwendung kann der Wirkstoff somit entweder als Tablette bzw. Kapsel bzw. Riegel in genau festgelegten Dosierungen dem körpereigenen Organismus zugeführt werden. Insbesondere können die betreffenden Tabletten bzw. Kapseln als Blisterverpackung vorgesehen sein.

Alternativ besteht auch die Möglichkeit, die Zusammensetzung in einer vom Nutzer selbst zu dosierenden Darreichungsform zur Verfügung zu stellen, beispielsweise als in Wasser aufzurührendes Pulver oder dergleichen.

## Patentansprüche

1. Zusammensetzung für ein Nahrungsergänzungsmittel, insbesondere für ein Sportler-Nahrungsergänzungsmittel, in flüssiger oder fester Form zur kontinuierlichen oder diskontinuierlichen Einnahme,
**dadurch gekennzeichnet, dass**
es als Bestandteil Heisteria Pallida enthält.

2. Zusammensetzung nach Anspruch 1,
**dadurch gekennzeichnet, dass**
Heisteria Pallida als vermahlenes Rindenpulver vorliegt.

3. Zusammensetzung nach Anspruch 1,
**dadurch gekennzeichnet, dass**
Heisteria Pallida als Extrakt vorliegt.

4. Zusammensetzung nach mindestens einem der vorhergehenden Ansprüche,
**dadurch gekennzeichnet, dass**
ein Vitamin, insbesondere Vitamin C enthalten ist.

5. Zusammensetzung nach mindestens einem der vorhergehenden Ansprüche,
**dadurch gekennzeichnet, dass**
es in einer fest dosierten Applikationsform vorgesehen ist.

6. Zusammensetzung nach Anspruch 5,
**dadurch gekennzeichnet, dass**
es in der Applikationsform einer Tablette oder Kapsel vorgesehen ist.

7. Zusammensetzung nach Anspruch 5 oder 6,
**dadurch gekennzeichnet, dass**
es als Blister vorgesehen ist.

8. Zusammensetzung nach mindestens einem der Ansprüche 1 - 5,
**dadurch gekennzeichnet, dass**
es in der Applikationsform eines Riegels vorgesehen ist.

9. Zusammensetzung nach mindestens einem der vorhergehenden Ansprüche,
**dadurch gekennzeichnet, dass**
je Verabreichungseinheit ein Anteil von mindestens 100 mg, vorzugsweise ein Anteil von 100 mg - 2000 mg, besonders vorzugsweise ein Anteil von 500 mg - 1500 mg, an Heisteria Pallida, insbesondere in Form der vermahlenen Rinde, vorgesehen ist.

10. Zusammensetzung nach mindestens einem der Ansprüche 2 - 9,
**dadurch gekennzeichnet, dass**
die Rinde einen Feinheitsgrad im Bereich von 100 - 1000 µm besitzt.

11. Zusammensetzung nach mindestens einem der vorhergehenden Ansprüche,
**dadurch gekennzeichnet, dass**
je Verabreichungseinheit mindestens 100 mg, vorzugsweise ein Anteil von 100 mg - 1000 mg an Vitamin vorgesehen ist.

12. Zusammensetzung nach mindestens einem der vorhergehenden Ansprüche,
**dadurch gekennzeichnet, dass**
bei der Anwendung durch Sportler eine diskontinuierliche Einnahme in einem definierten Zeitfenster vor der körperlichen Betätigung vorgesehen ist.

13. Verwendung von Heisteria Pallida als Bestandteil einer Zusammensetzung für ein Mittel, insbesondere ein Nahrungsergänzungsmittel, zur Steigerung der körperlichen Leistungsfähigkeit, insbesondere während sportlicher Betätigung.

14. Verwendung nach Anspruch 13,
**dadurch gekennzeichnet, dass**
die Einnahme von Heisteria Pallida vor sportlicher Betätigung in fest vorgegebenen Dosierungen erfolgt.
